# EUROPEAN PATENT APPLICATION

(11) **EP 3 327 104 A1**
(43) Date of publication of application: **30.05.2018**
(21) Application number: 16827308.4
(22) Date of filing: 21.07.2016
(51) Int. Cl.: C11B 1/04, C07D 311/62, A23L 2/52, A61K 8/42, A61K 31/352

(54) **METHOD FOR PRODUCING A CONCENTRATE OF POLYMER PROANTHOCYANIDINS WITH A CONCENTRATION OF MORE THAN 40% BY MEANS OF ENZYMATIC TREATMENTS AND WITHOUT USING ORGANIC SOLVENTS**

(30) Priority: 22.07.2015 ES 201531083
(71) Applicant: Consejo Superior De Investigaciones Científicas (CSIC), 28006 Madrid (ES)
(72) Inventor: SAURA CALIXTO, Fulgencio, 28040 Madrid (ES); PÉREZ JIMÉNEZ, Jara, 28040 Madrid (ES)
(74) Representative: Pons Ariño, Angel
(86) International application number: PCT/ES2016/070553
(87) International publication number: WO 2017/013299

(57) **Abstract**

The invention relates to a method for producing a concentrate of polymer proanthocyanidins with a concentration of more than 40%, characterised by the use of enzymatic treatments and by the absence of organic solvents and/or the extraction of organic solvents. This concentrate can be used as a functional ingredient, a food supplement, a cosmetic product and a pharmaceutical product.

## Description

The present invention relates to a method for producing a concentrate of polymer proanthocyanidins with a concentration of more than 40% by means of successive enzymatic treatments and without using organic solvents. Likewise, the invention relates to the concentrate produced by the method mentioned and to the use thereof as a food supplement, functional ingredient, pharmaceutical or cosmetic product.

### STATE OF THE ART

Proanthocyanidins are a type of polyphenolic compound consisting of flavan-3-ols and flavan-3,4-diols and that are found in certain plants, including some which are edible.

Traditionally, scientific and technological interest in proanthocyanidins has focused on the astringency and aroma that they provide to food and drinks. However, in the last two decades it has been shown that these compounds are bioavailable and have significant biological activity, having great potential for the preparation of functional ingredients, dietary supplements and drugs based on the their anti-inflammatory properties, properties of reducing cardiovascular risk and colorectal cancer and neuroprotective properties.

Proanthocyanidins in plants are in the form of oligomers and polymers. There is extensive bibliographic information on the composition and content of oligomers in different plants, collected in international databases such as the "USDA *Database for the Proanthocyanidin Content of Selected Food".*

Proanthocyanidins are obtained from different plant material by extraction methods with various solvents, complemented with purification methods to produce concentrates with a proanthocyanidin concentration of up to 25% (generally oligomeric), significant amounts of proanthocyanidins, mainly polymeric, remaining in the discarded residue. Therefore, it would be desirable to have a method capable of producing polymeric proanthocyanidins with a concentration of more than 40%, thus avoiding extraction processes with organic solvents and maximising the use of the original plant material.

### DESCRIPTION OF THE INVENTION

A first aspect of the present invention relates to a process for obtaining a concentrate of polymer proanthocyanidins with a concentration by weight of at least 40% preferably of at least 50%, more preferably of at least 60%, even more preferably of at least 70%, even more preferably of at least 80% and even more preferably of at least 90%, characterised in that it comprises successive enzyme treatment stages. Another characteristic of the present invention is that it does not use organic solvents in any stage or extraction use thereof.

Another aspect of the present invention relates to a concentrate of polymer proanthocyanidins with a concentration by weight of at least 40% preferably of at least 50%, more preferably of at least 60%, even more preferably at of least 70%, even more preferably of at least 80% and even more preferably of at least 90%, produced by the process described above.

Another aspect of the present invention relates to the use of the concentrate of polymer proanthocyanidins with a concentration by weight of at least 40% preferably of at least 50%, more preferably of at least 60%, even more preferably of at least 70%, even more preferably of at least 80% and even more preferably of at least 90%, as described above, as a food supplement, functional ingredient, or pharmaceutical or cosmetic product.

Another aspect of the present invention relates to a pharmaceutical composition containing a concentrate of polymer proanthocyanidins produced by the process defined above or a pharmaceutically acceptable salt thereof and one or more pharmaceutically acceptable excipients.

Another aspect of the present invention relates to the use of the pharmaceutical composition defined above for the manufacture of a pharmaceutical product intended for gastrointestinal health, including the inhibition of intestinal polyps and aberrant crypts as prevention and treatment elements of colon cancer. Likewise, it refers to the use in pharmaceutical products for reducing cardiovascular risk factors, mainly total cholesterol, LDL, triglycerides and percentage of body fat.

As such, the invention relates to the process for obtaining a concentrate of polymer proanthocyanidins with a concentration by weight of at least 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93 94, 95, 96, 97, 98 or 99%, as mentioned above, which comprises successive enzyme treatment stages without the use of organic solvents.

In one embodiment, the invention relates to the process described above, wherein the concentration by weight of the concentrate of polymer proanthocyanidins is of at least 40%.

In one embodiment, the invention relates to the process described above, wherein the concentration by weight of the concentrate of polymer proanthocyanidins is of at least 50%.

In one embodiment, the invention relates to the process described above, wherein the concentration by weight of the concentrate of polymer proanthocyanidins is of at least 60%.

In one embodiment, the invention relates to the process described above, wherein the concentration by weight of the concentrate of polymer proanthocyanidins is of at least 70%.

In another embodiment, the invention relates to the process described above, wherein the concentration by weight of the concentrate of polymer proanthocyanidins is of at least 80%.

In another embodiment, the invention relates to the process described above, wherein the concentration by weight of the concentrate of polymer proanthocyanidins is of at least 90%.

In another embodiment, the invention relates to the process described above, wherein the polymer proanthocyanidins consist of (epi)catechin, (epi)gallocatechin and (epi)catechin-3-O-gallate monomers.

In another embodiment, the invention relates to the process described above, wherein the polymer proanthocyanidins are of formula I: 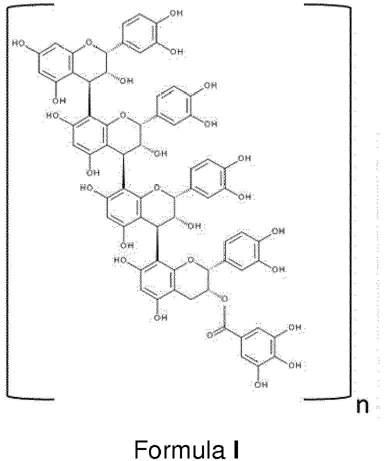 wherein n represents a number between 11 and 250, preferably between 11 and 150.

In another embodiment, the invention relates to the process described above, wherein the enzymes are selected from proteases, hemicellulases and cellulases.

In another embodiment, the invention relates to the process described above, wherein the use of the enzymes is by sequential action, and preferably wherein the use of the enzymes is by sequential action by means of a first protease treatment and a subsequent hemicellulase and cellulase treatment.

In another embodiment, the invention relates to the process described above, wherein:
the enzymes are selected from proteases, hemicellulases and cellulases; and
the use thereof is by sequential action, and preferably wherein the use of the enzymes is by sequential action by means of a first protease treatment and a subsequent hemicellulase and cellulase treatment.

In another embodiment, the invention relates to the process described above, wherein the proteases are selected from proteases from *Bacillus licheniformis* type VIII.

In another embodiment, the invention relates to the process described above, characterised for being produced from plant material.

In another embodiment, the invention relates to the process described above, wherein the plant material is selected from persimmon, carob, banana, grape, cocoa or combinations thereof.

As the person skilled in the art knows, factors such as the type of variety of selected plant material, the environment in which it develops, or the harvest time can have a minimum deviation effect on the concentration of polymer proanthocyanidins produced by the process of the invention.

The scope of the invention includes any combination of plant material, regardless of whether or not they are included in the present document, which when used in the process of the invention, they achieve a concentration by weight of the concentrate of polymer proanthocyanidins of at least 40%, preferably of at least 50%, more preferably of at least 60%, even more preferably of at least 70%, even more preferably of at least 80% and even more preferably of at least 90%, as described in this document.

In another embodiment, the invention relates to the process described above, wherein the plant material is a combination of cocoa with persimmon and/or carob and/or red grape and that produces a final concentration by weight of the concentrate of polymer proanthocyanidins of at least 70%.

In another embodiment, the invention relates to the process described above, characterised in that the plant material is previously treated with an aqueous medium in order to produce a supernatant (containing sugars and compounds of low molecular weight) that is removed, thus producing a solid residue containing polymer proanthocyanidins. Carbohydrate polymers and proteins and other compounds are removed by means of enzymatic treatments on this residue, thus producing a concentrate of polymer proanthocyanidins having differentiating characteristics with regards to the current proanthocyanidins extracts: higher molecular weight of the proanthocyanidins, lack of organic solvents in the production, and greater use of the original plant material.

In another embodiment, the invention relates to the process described above, wherein the residue resulting from the enzymatic treatment is subjected to a drying stage.

In another embodiment, the invention relates to the process described above, wherein the drying is carried out by freeze-drying or vacuum drying.

In another embodiment, the invention relates to the process described above, wherein the concentrate of polymer proanthocyanidins produced is subjected to grinding in order to produce a particle size from nanometres to 250 mm.

The present invention relates to a food ingredient comprising the product of the invention and one or more food additives with acceptable technological use, that is, that they are compatible with the product of the invention and are not harmful to those who take said product, and add functional, sensory, stability or technological improvements. This ingredient can be incorporated in different doses into different food matrices (beverages, baked goods, meat and fish preparations, spreadable products, dairy products, etc.), giving rise to a new product.

The present invention also relates to a cosmetic product comprising the product of the invention and one or more cosmetically acceptable excipients, that is, that they are compatible with the product of the invention and are not harmful to those who take said product, and add functional, sensory, stability or technological improvements. This cosmetic product can have any of the usual forms of presentation of these products: cream, ointment, lotion, etc.

The present invention also relates to a food supplement comprising the product of the invention and one or more pharmaceutically acceptable excipients, that is, that they are compatible with the product of the invention and are not harmful to those who take said supplement, and add functional, sensory, stability or technological improvements. This food supplement can have any of the usual forms of presentation of these products: sachets, tablets, capsules, etc.

The present invention also relates to a pharmaceutical composition comprising the compound of the invention alone or with other active compounds (or a pharmaceutically acceptable salt o solvate thereof) and one or more pharmaceutically acceptable excipients. The excipients must be "acceptable" in the sense that they are compatible with the other ingredients of the composition and are not harmful to those who take said composition.

Throughout the invention, the term "sequential action" refers to the consecutive use of the enzymes of the process of the invention. Preferably, such that a protease treatment is initially carried out and subsequently a hemicellulase and cellulase treatment.

The compounds of the present invention can be administered in the form of any pharmaceutical formulation, the nature of which, as is known, depends on the nature of the active ingredient and the route of administration thereof. The ingredient can be used in any route of administration, for example oral, parenteral, nasal, ocular, rectal and topical.

The solid compositions for oral administration include tablets, granules and capsules. In any case, the manufacturing method is based on a simple mixing, dry granulation or humid granulation of the active ingredient with excipients. These excipients can be, for example, diluents such as lactose, microcrystalline cellulose, mannitol or calcium hydrogen phosphate; binder agents such as, for example, starch, gelatin or polyvinylpyrrolidone; disintegrants such as sodium carboxymethyl starch or croscarmellose sodium; and lubricant agents such as, for example, magnesium stearate, stearic acid or talc. The tablets can also be coated with suitable excipients and by means of known techniques with the aim of delaying the disintegration and absorption thereof in the gastrointestinal tract and thus achieve a sustained action for a longer period of time, or simply to improve the organoleptic properties or stability thereof. The active ingredient can also be incorporated by coating on inert pellets by means of the use of natural or synthetic filmogenic polymers. It is also possible to make soft gelatin capsules, wherein the active ingredient is mixed with water or with an oily medium, for example coconut oil, liquid paraffin or olive oil.

Powders and granules can be produced for the preparation of oral suspensions by adding water, mixing the active ingredient with dispersing or wetting agents; suspending agents and preservatives. Other excipients can also be added, for example sweeteners, flavourings and colourants.

As liquid forms for oral administration, emulsions, solutions, suspensions, syrups and elixir can be included, which contain commonly used inert diluents, such as distilled water, ethanol, sorbitol, glycerol, polyethylene glycols (macrogols) and propylene glycol. Said compositions can also contain processing aids such as wetting agents, suspending agents, sweeteners, flavourings, preservatives and pH regulators.

Injectable preparations, according to the present invention, for parenteral administration comprise sterile solutions, suspensions or emulsions in an aqueous or non-aqueous solvent such as propylene glycol, polyethylene glycol or plant oils. These compositions can also contain processing aids, such as wetting agents, emulsifiers, dispersing agents and preservatives. They could be sterilised by any of the known methods or prepared as sterile solid compositions that can be dissolved in water or any other sterile injectable medium immediately before use. It is also possible to use sterile raw materials and keep them in these conditions throughout the entire manufacturing process.

For rectal administration, the active ingredient can be preferably formulated as an oil-based suppository, such as, for example, plant oils or solid semisynthetic glycerides, or hydrophilic-based suppository such as polyethylene glycols (macrogols).

The compounds of the invention can also be formulated for the topical application thereof to treat pathologies in areas or organs that are accessible through this route, such as eyes, skin and intestinal tract. Formulations include creams, lotions, gels, powders, solutions and patches wherein the compound is dispersed or dissolved in suitable excipients.

For nasal administration or by inhalation, the compound can be formulated in the form of aerosols from where it is conveniently released with the use of suitable propellants.

The dosage and frequency of the dose vary depending on the nature and severity of the illness to be treated, the age, general condition and weight of the patient, as well as that of the particular compound administered and the route of administration, among other factors. By way of example, a suitable dosage range varies between approximately 0.01 mg/Kg and approximately 100 mg/Kg per day, which can be administered as a single dose or in several doses.

Throughout the description and the claims, the word "comprises" and its variants are not intended to exclude other technical characteristics, additives, components or steps. For those skilled in the art, other objects, advantages and characteristics of the invention may be deduced from both the description and the practical use of the invention. The following examples are provided by way of illustration and are not intended to be limiting of the present invention.

### EXAMPLES

The invention is illustrated below by means of tests carried out by the inventors which reveal the effectiveness of the product of the invention.

The enzymatic treatments and the experimental conditions thereof were selected after many tests with different types of enzymes and combinations thereof.

In this way, the following enzymes were discarded for the use thereof in the process of the present invention:
1) Pepsin. Pepsin from porcine gastric mucosa (P700 Sigma-Aldrich) was used, and this method was carried out: adding 10 L of HCl-KCl buffer (HCl-KCI buffer 0.2M: preparing 14.91 g of KCI/ 1000 ml distilled water. Furthermore, 15.56 g HCl 37%/ 1000 ml of distilled water. Mixing 250 ml of KCL with 150 ml of HCL and adding 500 of distilled water. Adjusting pH to 1.5 and bringing to the volume of 1 litre) and adjusting the pH to 1.5. Adding 200 mL of pepsin (300 mg/ml HCl-KCl buffer), and incubating for 1 hr at 40ºC.
2) Pancreatin. Pancreatin from porcine pancreas (P7545 Sigma-Aldrich) was used: adding 4.5 L of phosphate buffer pH 7.5 (Phosphate buffer 0.1M: preparing 13.8 g of sodium phosphate in 700 ml of distilled water. Furthermore, 20.41 g of NaOH in 500 ml of distilled water. Adding 70 ml NaOH 1N, adjusting pH to 7.5 and bringing to the volume of 1 litre) and adjusting the sample to this pH. Adding 1 L of pancreatin (5 mg/ml phosphate buffer pH 7.5) and incubating for 6 hrs at 37ºC.
3) Viscozyme. A cellulolytic enzymatic complex from *Aspergillus sp.* (V2010 Sigma-Aldrich) was used, 10 L of acetate buffer 0.1M was added (acetate buffer 0.1M: mixing 3.33 g sodium acetate and 900 mL of distilled water. Adjusting pH to 4.6 and bringing to the volume of 1L with distilled water), adjusting pH to 4.6 and adding 20 mL of Viscozyme (commercial solution) and incubating for 12 hrs at 50ºC.
4) Xylanase. Xylanase from *Thermomyces lanuginosus* (X2753 Sigma-Aldrich) was used. Adding 10 L of acetate buffer 0.1 M, adjusting the pH to 4.6, adding 1 L of xylanase (6 mg/mL acetate buffer 0.1M) and incubating for 16 hrs at 75ºC.

As such, the use of these three enzymes in the tests carried out for the invention has been ruled out due to having a concentration of polymer proanthocyanidins to the order of 40% lower (in the case of viscozyme and xylanase) and of 50% lower (in the case of pepsin and pancreatin) than that achieved with the enzymes selected to carry out the invention and that are detailed below.

### Example 1: producing the concentrate of polymer proanthocyanidins.

### Production based on carob (carob pod).

### 1) Preparation of plant material.

The carob pods are used. Firstly, the seeds (which are currently used to industrially produce locust bean gum) are extracted and discarded and the pods are ground, resulting in a carob flour (hereinafter, original flour).

The flour is homogenised in an aqueous medium with a *Polytron* (or similar device) using a suitable proportion of water (15 L water/kg original flour). The homogenate is stirred (30 minutes at a temperature of less than 60ºC) in order to dissolve sugars and other soluble solids. Then, it is centrifuged (10 minutes at 3000 *g*)*,* the supernatant is discarded and the resulting solid residue is the starting material for producing the concentrate of polymer proanthocyanidins.

### 2) Producing the concentrate of polymer proanthocyanidins.

It requires successive enzymatic treatments that aim to eliminate the insoluble and polymeric compounds other than polymer proanthocyanidins contained in the residue from centrifugation.

### 2.1 Protease treatment

An aqueous solution of NaOH 0.275 M (10 L/kg original flour) is added to the residue from 1), the pH is adjusted to 7.5 and it is homogenised in a similar way to that described in 1). 100 mL of protease from *Bacillus licheniformis* (phosphate buffer solution 0.08M pH 6.0) is added. It is incubated for 35 mins at 60ºC with constant stirring. It is centrifuged (10 minutes at 3000 *g*)*,* the supernatant is discarded and the residue is used in the following stage.

### 2.2 Combined cellulase and hemicellulase treatment

An acetate buffer pH 4.6 0.1M (5L/kg original flour) is added to the residue from 2.1, the pH is adjusted to 4.6 and it is homogenised. 500 mL of cellulase (activity 2 u/mg) and 500 mL of hemicellulase (activity 0.3 u/mg) are added, both in the buffer solution pH 4.6. It is incubated for 16 hrs at 43ºC with constant stirring. It is centrifuged (10 minutes at 3000 g), the supernatant is discarded and the residue is used in the following stage.

### 2.3 Drying and grinding

The residue from 2.2 is dried at a low temperature (<60ºC) using common technology (freeze-drying or vacuum drying or spray-drying, etc.). It is then subjected to grinding in order to produce a particle size that is suitable according to the intended application, also using common technology (centrifugal mill or others, micronizer mill, formation of nanoparticles, etc.).

### 3) Resulting product

It is a dry powder, with a concentration of polymer proanthocyanidins of more than 90%.

The content is determined according to the method described by Zurita (see Int. J. Food Sci. Nutr., "Improved procedure to determine non-extractable polymeric proanthocyanidins in plant foods", 63, 936-39)

### Production based on persimmon.

The same method is used as in the case of the carob, but in this case the plant material used is the pulp and skin of the persimmon (pitted persimmon). A dry powder is produced with a concentration of polymer proanthocyanidins of more than 80%.

### Production based on banana

The same method is used as in the case of the carob, but in this case the plant material used is the skin of the banana. A dry powder is produced with a concentration of polymer proanthocyanidins of more than 70%.

### Production based on red grapes (red grape pomace)

The same method is used as in the case of the carob, but in this case the plant material used is the red grape pomace. A dry powder is produced with a concentration of polymer proanthocyanidins of more than 90%.

### Production based on cocoa

The same method is used as in the case of the carob, but in this case the plant material used is non-fat cocoa. A dry powder is produced with a concentration of polymer proanthocyanidins of more than 40%.

### Production based on a combination of cocoa and other plant materials

The same method is used as in the case of carob, but in this case the plant material used is a combination of non-fat cocoa with grape pomace, or with carob pod or with persimmon pulp and skin, in proportions that enable a dry powder to be produced with a concentration of polymer proanthocyanidins of at least 70%, 80% or 90%.

### Example 2: Use of the polymer proanthocyanidins as a food supplement or functional ingredient

In a previous study, a grape product containing 15% of polymer proanthocyanidins was provided through one-dose sachets (7.5 g of product), the content of which was dissolved in water for the consumption thereof. After daily consumption of this product for 16 weeks, positive results were observed in hypercholesterolemic subjects in different parameters: total cholesterol, LDL cholesterol, triglycerides, % of body fat, blood pressure (Pérez-Jiménez et al., Nutr., 24, 2008, 646-53). Numerous studies on animals with grape proanthocyanidins have observed these effects and have explained the molecular mechanisms involved (Bladé et al., Molec. Nutr. Food Res., 54, 2010, 37-59; Decordé et al., Molec. Nutr. Food Res., 53, 2009, 659-66; Kruger et al., Food Res. Int., 59, 2014, 41-52); similarly, these effects have been observed for persimmon proanthocyanidins of high molecular weight (Zou et al., Food Res. Intl., 48, 2012, 970-77). Similarly, the grape proanthocyanidins have shown, in different animal models, positive effects in relation to the metabolism of carbohydrates, including blood glucose, insulinemia and other related markers (Castell-Auvi et al., J. Nutr. Biochem., 23, 2012, 1565-72; Mesproom et al., Br. J. Nutr., 106, 2011, 1173-81). It is to be expected that the concentrate whose production is described in this invention, with a much higher concentration of polymer proanthocyanidins, produces more pronounced results and/or with smaller doses.

### Example 3: Use of the polymer proanthocyanidins as a cosmetic product

It has been described that grape proanthocyanidins are capable of inhibiting the oxidation caused by UV rays in epidermis cell cultures (keratinocytes) (Mantena et al. Free Radical Biol. Med., 40, 2006, 1603-14). Similar effects were observed in UVB-irradiated mice, after the topical application of a grape proanthocyanidin extract (Filip et al. Food Chemical Toxicol., 2013). It is to be expected that the concentrate whose production is described in this invention, with a much higher concentration of polymer proanthocyanidins, produces more pronounced results and/or with smaller doses.

### Example 4: Pharmaceutical composition containing polymer proanthocyanidins to prevent cardio-metabolic risk

As indicated in Example 2, the prolonged consumption of polymer proanthocyanidins has given rise to beneficial effects on different cardio-metabolic risk markers. This is due to different properties of proanthocyanidins, among them, the known anti-inflammatory effects thereof (Kruger et al., Food Res. Int., 59, 2014, 41-52; Mena et al., IUBMB Life, 66, 2014, 745-58). It is to be expected that the greater concentration of these compounds in the product of the present invention enable these effects to be reached at lower doses and could therefore be incorporated into pharmaceutical formulations, alone or in combination with other active compounds incorporated in common clinical practice.

### Example 5: Use of the pharmaceutical composition for gastrointestinal health, including the inhibition of intestinal polyps and aberrant crypts as prevention and treatment elements of colon cancer

Previous studies have shown that the proanthocyanidins of different products derived from grape have beneficial effects in relation to different parameters of gastrointestinal health: regulating effects in ulcerative colitis (Wang et al., Intl. Inmunopharmacol., 10,2011, 1620-27), increase in caecal antioxidant capacity (Goñi and Serrano, J Sci Food Agric 85,1877-1881), reduction of lipid oxidation in colonic mucosa (López-Oliva et al., Br J Nutr 14, 2013, 4-16), overexpression of detoxifying enzymes (López-Oliva et al. Nutr. Res., 26, 2006, 653-68; López-Oliva et al., Br JNutr 14, 2013, 4-16), stimulation of *Lactobacillus* growth (Pozuelo et al., J Food Sci 77, 2012, 59-6). It is particularly noteworthy that these compounds have shown capacity to reduce the intestinal crypts in healthy rats (López-Oliva et al. Nutr. Res., 26, 2006, 653-68), as well as the number and size of the intestinal polyps in an animal model with confirmed colon cancer, the mice being Apc^{Min+/-} (Sánchez-Tena et al. Carcinog., 34, 2013, 1881-88; Velmuragan et al., Neoplasia, 12, 2010, 95-102), having identified some of the molecular mechanisms responsible for these effects (Kaur et al., Mol. Carcinog., 50, 2011, 553-62; Roy et al., Neoplasia, 7, 2005, 24-36). It is to be expected that the concentrate whose production is described in this invention, with a much higher concentration of polymer proanthocyanidins, produces more pronounced results and/or with smaller doses.

## Claims

1. A process for obtaining a concentrate of polymer proanthocyanidins with a concentration by weight of at least 40%, **characterised in that** it comprises successive enzyme treatment stages.

2. The process according to claim 1, wherein the concentration by weight of the polymer proanthocyanidins is of at least 50%.

3. The process according to claim 2, wherein the concentration by weight of the polymer proanthocyanidins is of at least 60%.

4. The process according to claim 3, wherein the concentration by weight of the polymer proanthocyanidins is of at least 70%.

5. The process according to claim 4, wherein the concentration by weight of the polymer proanthocyanidins is of at least 80%.

6. The process according to claim 5, wherein the concentration by weight of the polymer proanthocyanidins is of at least 90%.

7. The process according to any of claims 1 to 6, wherein the polymer proanthocyanidins consist of flavanols.

8. The process according to claim 7, wherein the flavanol monomers are selected from (epi)catechin, (epi)gallocatechin and (epi)catechin-3-O-gallate.

9. The process according to any of claims 1 to 6, wherein the polymer proanthocyanidins are of the formula I: 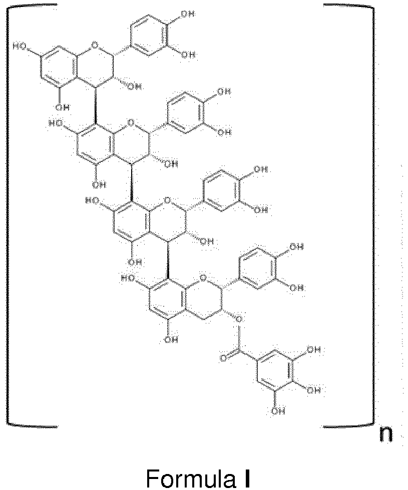 wherein n represents a number between 11 and 250.

10. The process according to claim 9, wherein the polymer proanthocyanidins are of formula I and n represents a number between 11 and 150.

11. The process according to any of claims 1 to 10, wherein the enzymes are selected from proteases, hemicellulases and cellulases.

12. The process according to claim 11, wherein the proteases are selected from *Bacillus licheniformis* type VIII.

13. The process according to any of claims 1 to 12, **characterised in that** it is produced from plant material.

14. The process according to claim 13, wherein the plant material is selected from persimmon, carob, banana, grape, cocoa or the combinations thereof.

15. The process according to any of claims 13 or 14, **characterised in that** the plant material is previously treated in an aqueous medium that is centrifuged and decanted in order to obtain the solid residue contained in the concentrate of proanthocyanidins.

16. The process according to any of claims 1 to 15, wherein the residue resulting from the enzymatic treatment is subjected to a drying stage.

17. The process according to claim 16, wherein the drying is carried out by freeze-drying or vacuum drying.

18. The process according to any of claims 1 to 17, wherein the concentrate of polymer proanthocyanidins obtained is subjected to grinding in order to obtain a particle size from nanometres to 250 mm.

19. Concentrate of polymer proanthocyanidins with a concentration by weight of at least 40% produced by the method described in any of claims 1 to 18.

20. The concentrate according to claim 19, wherein the concentration by weight of polymer proanthocyanidins is of at least 50%.

21. The concentrate according to claim 20, wherein the concentration by weight of the polymer proanthocyanidins is of at least 60%.

22. The concentrate according to claim 21, wherein the concentration by weight of the polymer proanthocyanidins is of at least 70%.

23. The concentrate according to claim 22, wherein the concentration by weight of the polymer proanthocyanidins is of at least 80%.

24. The concentrate according to claim 23, wherein the concentration by weight of the polymer proanthocyanidins is of at least 90%.

25. Use of the concentrate of polymer proanthocyanidins as defined in claims 19 to 24, as a food supplement, functional ingredient or cosmetic product.

26. A pharmaceutical composition containing the concentrate of polymer proanthocyanidins as defined in claims 19 to 24, alone or as part of a formulation with other active compounds, or a pharmaceutically acceptable salt thereof and one or more pharmaceutically acceptable excipients.

27. Use of the composition as defined in claim 26 for the manufacture of a pharmaceutical product for gastrointestinal health, including the inhibition of intestinal polyps and aberrant crypts as prevention and treatment elements of colon cancer.

28. Use of the composition as defined in claim 26 for the manufacture of a pharmaceutical preparation for reducing cardiovascular risk factors, such as total cholesterol, LDL, triglycerides and percentage of body fat, among others.
